# EUROPEAN PATENT APPLICATION

(11) **EP 1 943 950 A1**
(43) Date of publication of application: **16.07.2008**
(21) Application number: 08100230.5
(22) Date of filing: 09.01.2008
(51) Int. Cl.: A61B 6/00, A61B 6/03

(54) **X-ray detector arrangement**

(30) Priority: 09.01.2007 US 621206
(71) Applicant: General Electric Company, Schenectady, NY 12345 (US)
(72) Inventor: Luusua, Jarmo, FI-01300, Vantaa (FI)
(74) Representative: Illingworth-Law, William Illingworth

(57) **Abstract**

X-ray detector arrangement comprising a detector (1) and an anti-scatter grid (6), whereby the anti-scatter grid is arranged to guide the x-rays in a desired way. The arrangement further comprises a flat box (2) having two flat surfaces (3,4) spaced from a distance to each other, and edge elements (5) by which the flat surfaces are connected to each other. The anti-scatter grid (6) is integrated to one flat surface of the flat box, and the flat box are provided with a slot (7) at least on one edge of the box to enable insertion of the detector (1) between the two flat surfaces (3,4) of the box (2).

## Description

### BACKGROUND

The invention relates to an x-ray detector arrangement to be used for example in clinical use especially with a mobile x-ray unit.

The x-detector arrangements described above are very common for example in hospitals. Normally in clinical use a tethered portable detector is used in conjunction with a separate x-ray anti-scatter grid attached on top of the detector. The anti-scatter grid is needed to prevent the x-rays from scattering from tissue in x-ray path to the detector.

In the prior art solutions the x-ray grid is attached on top of the detector. The weight that the user has to lift and handle is thus the weight of the detector plus the grid. The detector with a grid attached on top of the detector described above weighs typically about 6 kg. The x-ray detector arrangement, i.e. the detector and the grid has to be pushed for example under the bed-bound patient to be examined. In other words the detector arrangement, i.e. the detector and the grid attached to the detector must be pushed under the patient so that the x-ray detector is moved between the patient and the mattress on which the patient is lying. Pushing the detector with the grid under the patient is difficult and pulling out the detector is similarly difficult.

As told above the procedure described above is in practice very often difficult because at least two nurses/x-ray technologists are required to carry out said procedure. This is due to the fact that considerable physical force is required to lift the patient and to handle a heavy detector arrangement at the same time. While pushing/pulling the detector arrangement under the patient in the way as described above there is a risk of damage to the detector tether if the strain relief of the detector tether is used as a handle.

The problems described above occur both in digital imaging mode, including CR-plate, and in traditional film based mode. In the traditional film based mode it is known to use an outer sheath in connection with the x-ray film cassette used. Said outer sheath is made of cloth material. The material forming the outer sleeve is locked around the cassette by using a Velcro slip or the like. This arrangement is however not so practical especially in connection with digital technique using relatively heavy detector arrangement. Especially cleaning of the sheath is difficult due to the fact of a cloth material being able to be impregnated with all bodily fluids that may carry different bacteria or viruses the sheath comes in contact with.

### SUMMARY

The object of the invention is to obtain an x-ray detector arrangement with which the disadvantages of the prior art can be eliminated. This is obtained with the present invention. The present invention is characterized in that the arrangement comprises a flat box having two flat surfaces spaced from a distance to each other, and edge elements by which the flat surfaces are connected to each other, the anti scatter x-ray grid being integrated to one flat surface of the flat box, and the flat box being provided with a slot at least on one edge of the box to enable insertion of the detector between the two flat surfaces of the box.

An advantage of the invention is that clinical work is much easier when compared to the prior art. Easier clinical work is obtained because in the invention the box, which is provided with the grid weighs approximately 1 kg and said relatively light component can be first inserted under the patient. This procedure is much easier than inserting heavy arrangement of the prior art under the patient. After the grid box has been correctly placed under the patient the detector can be relatively easily inserted into the grid box, and therefore risks of damages to the detector are eliminated. An advantage of the invention is also in that the invention can be materialized rather simply, i.e. manufacturing costs are rather low.

### BRIEF DESCRIPTION OF THE DRAWINGS

In the following the invention will be described in more detail by way of example only, with reference made to the attached drawings in which:
Figure 1 shows one embodiment of the x-ray detector arrangement in a perspective view, and
Figure 2 shows principally the x-ray detector arrangement shown in Figure 1 in clinical use.

### DETAILED DESCRIPTION OF THE DRAWINGS

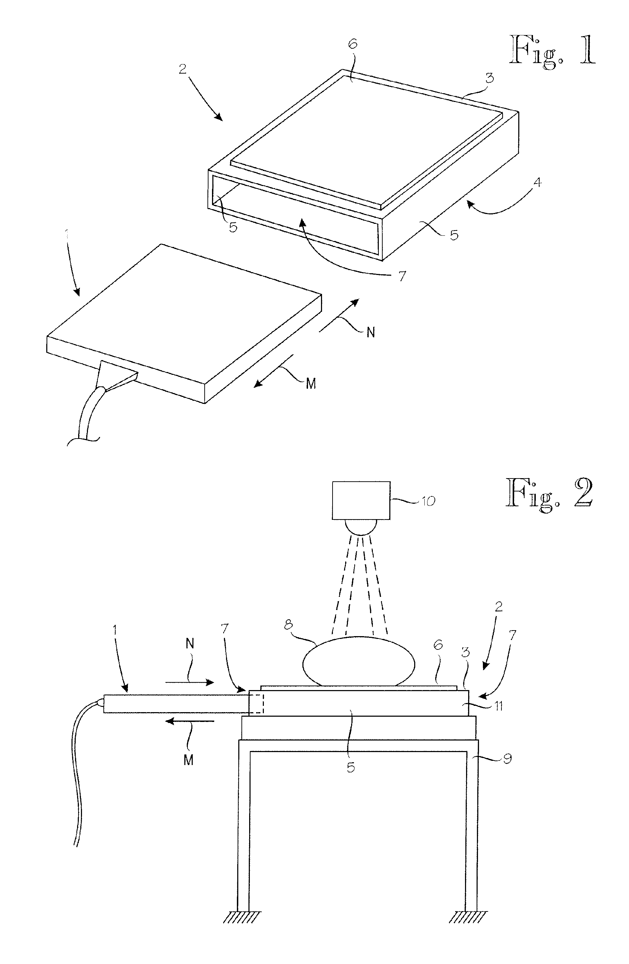

Figure 1 shows one embodiment of the x-ray arrangement of the invention. The basic parts of the invention are shown so that reference number 1 shows the detector and reference number 2 shows a flat box, which is an essential part of the invention.

According to the invention the x-ray arrangement comprises the detector 1 and the flat box 2 so that the flat box 2 has two flat surfaces 3, 4 spaced from a distance to each other, and edge elements 5 by which the flat surfaces are connected to each other. The anti-scatter grid 6 guiding x-rays has been integrated to one flat surface of the flat box 2. The flat box 2 is further provided with a slot 7 at least on one edge of the box to enable insertion of the detector 1 between the two flat surfaces of the box 2.

Figure 2 shows the embodiment of the invention shown in Figure 1 in use. Figure 2 shows however also a patient 8 lying on a bed 9. Figure 2 shows also schematically an x-ray machine 10. Reference number 11 shows a mattress on which the patient is lying.

The invention can be used basically as follows. The invention is suited especially well in connection with mobile x-ray devices, i.e. with x-ray devices, which can be moved to the patient in order to get images needed. First the x-ray machine 10 is moved to the patient and then the flat box is located under the patient. In this connection it must be noted that the flat box can be easily pushed under the patient because the flat box weighs only about 1 kg. It must also be noted that the flat box can be made as a strong construction and in the flat box there are no mechanically weak electrical connections and the like, which cause problems if prior art solutions are used.

As told above the flat box is provided a slot 7 at least on one edge of the box in order to enable insertion of the detector between the two flat surfaces 3, 4 of the flat box 2. In other words the box 2 and the slot 7 have been dimensioned so that the detector 1 can be inserted into the flat box 2 as shown by arrow N in Figures 1 and 2.

Figure 2 shows a situation in which the detector is pushed into the flat box placed under the patient 8. The flat box 2 has been dimensioned so that the box is big enough to accept the detector snugly and allowing it to be pushed in and out easily. The inside surfaces of the box may also be provided with a low friction material in order to make pushing and pulling the detector easy.

After the images needed have been taken the detector is pulled out from the box as shown by arrow M in Figures 1 and 2.

The procedure, which must be carried out, i.e. insertion of the detector and the grid under the patient to be treated in considerably easier with the arrangement of the invention when compared to the procedure needed in connection with the prior art. When using the invention no excessive force is needed but the flat box can be pushed under the patient rather easily and insertion of the detector into the flat box is extremely easy task. Removal of the equipment is also as easy procedure as the insertion step.

In the embodiment show in the figures the edge elements 5 are edge walls extending over the edge lengths of the flat surfaces 3, 4. Said edge elements can also be formed by using for example narrow elements in the corners of the flat surfaces 3, 4.

The slot 7 can be also arranged for example on two edges of the flat box. Said two slots can be provided for example at opposite edges of the flat box as shown with reference numerals 7 in Figure 2. The flat box can also be formed so that there is a slot on every edge side of the box. This embodiment can be obtained for example by using narrow elements as edge elements 5 in the corners of the flat surfaces 3, 4.

The flat box can be made of any appropriate material in order to obtain a rigid and self-supporting construction. An example of the materials, which can be used carbon fibre material can be mentioned.

The flat box can also be provided with rounded corners in order to enable smooth gliding under the patient. The flat box can also be provided with appropriate handles in order to make handling of the box easier.

The invention is by no means limited to the embodiments described above but different details of the invention can be varied within the scope of the annexed claims.

## Claims

1. X-ray detector arrangement comprising a detector (1) and an anti-scatter x-ray grid (6), the anti-scatter grid being arranged to guide the x-rays in a desired way, **characterized in that** the arrangement further comprises a flat box (2) having two flat surfaces (3,4) spaced from a distance to each other, and edge elements (5) by which the flat surfaces are connected to each other, the anti-scatter grid (6) being integrated to one flat surface of the flat box, and the flat box being provided with a slot (7) at least on one edge of the box to enable insertion of the detector (1) between the two flat surfaces (3,4) of the box (2).

2. X-ray detector arrangement according to claim 1, **characterized in that** the slot (7) is provided at least at two edges of the flat box (2).

3. X-ray detector arrangement according to claim 2, **characterized in that** the slots (7) are provided at opposite edges of the flat box (2).

4. X-ray detector arrangement according to any one of the preceding claims, **characterized in that** the flat box (2) is a self-supporting structure.

5. X-ray detector arrangement according to claim 4, **characterized in that** the flat box (2) is made of carbon fibre material.

6. X-ray detector arrangement according to any one of the preceding claims, **characterized in that** the flat box (2) is provided with rounded corners.

7. X-ray detector arrangement according to any one of the preceding claims, **characterized in that** the arrangement is designed for use with a mobile x-ray unit (10).
